# EUROPEAN PATENT APPLICATION

(11) **EP 1 783 122 A1**
(43) Date of publication of application: **09.05.2007**
(21) Application number: 05765585.4
(22) Date of filing: 07.07.2005
(51) Int. Cl.: C07D 309/10, C07H 7/04, C07H 15/04, C07C 43/225, C07C 49/223

(54) **PROCESS FOR PRODUCTION OF AZULENE DERIVATIVES AND INTERMEDIATES FOR THE SYNTHESIS OF THE SAME**

(30) Priority: 08.07.2004 JP 2004202437
(71) Applicant: Astellas Pharma Inc., Tokyo 103-8411 (JP); Kotobuki Pharmaceutical Co., Ltd., Hanishina-gun, Nagano 389-0697 (JP)
(72) Inventor: TOMIYAMA, Hiroshi, KOTOBUKI PHARMACEUTICAL CO.,LTD, Hanishina-gun, Nagano 3890697 (JP); YOKOTA, Masayuki, KOTOBUKI PHARMACEUTICAL CO., LTD, Hanishina-gun, Nagano 3890697 (JP); NODA, Atsushi, KOTOBUKI PHARMACEUTICAL CO., LTD., Hanishina-gun, Nagano 3890697 (JP); KOBAYASHI, Y., KOTOBUKI PHARMACEUTICAL CO., LTD., Hanishina-gun, Nagano 3890697 (JP); OGASAWARA, Junko, KOTOBUKI PHARMACEUTICAL CO., LTD, Hanishina-gun, Nagano 3890697 (JP); HAYASHI, Yasumasa, ASTELLAS PHARMA INC., Chuo-ku, Tokyo 1038411 (JP); INAKOSHI, Masatoshi, ASTELLAS PHARMA INC., Chuo-ku, Tokyo 1038411 (JP); NAKAMURA, Hirofumi, ASTELLAS PHARMA INC., Chuo-ku, Tokyo 1038411 (JP); KOIDE, Tokuo, ASTELLAS PHARMA INC., Chuo-ku, Tokyo 1038411 (JP); SAKAMOTO, Kenichiro, ASTELLAS PHARMA INC., Chuo-ku, Tokyo 1038411 (JP); YAMASHITA, Yohei, ASTELLAS PHARMA INC., Chuo-ku, Tokyo 1038411 (JP); MIYAFUJI, Akio, ASTELLAS PHARMA INC., Chuo-ku, Tokyo 1038411 (JP); SUZUKI, Takayuki, ASTELLAS PHARMA INC., Chuo-ku, Tokyo 1038411 (JP); KAWANO, Noriyuki, ASTELLAS PHARMA INC., Chuo-ku, Tokyo 1038411 (JP); MIYATA, Junji, ASTELLAS PHARMA INC., Chuo-ku, Tokyo 1038411 (JP); IMAMURA, Masakazu, ASTELLAS PHARMA INC., Chuo-ku, Tokyo 1038411 (JP); SUGANE, Takashi, ASTELLAS PHARMA INC., Chuo-ku, Tokyo 1038411 (JP)
(74) Representative: HOFFMANN EITLE
(86) International application number: PCT/JP2005/012577
(87) International publication number: WO 2006/006496

(57) **Abstract**

A process for producing an azulene derivative useful as a Na⁺-glucose cotransporter inhibitor, which is high in yield, is simple in operation, is low in cost, is suited for environmental protection, and is advantageous industrially, the process being **characterized by** reducing and deprotecting at least one compound selected from penta-acyl compounds and tetra-acyl compounds or salts thereof to obtain a C-glycoside compound; and a useful intermediate for synthesis of such an azulene derivative, obtained in the course of the above process.

## Description

### Technical Field

The present invention relates to a process for producing azulene derivatives useful as a Na⁺-glucose cotransporter inhibitor, as well as to intermediates for synthesis of such azulene derivatives. More particularly, the present invention relates to a process for producing azulene derivatives, which are high in yield, is simple in operation, is low in cost, is suited for environmental protection, and is advantageous industrially, as well as to useful intermediates for synthesis of such azulene derivatives, obtained in the course of the process.

### Background Art

The azulene derivative represented by the following structural formula (6) and salt thereof are known to have an effect of inhibiting a Na⁺-glucose cotransporter and be useful as a therapeutic agent for diabetes, etc. [see WO 04/13118 Pamphlet (hereinafter referred to as Patent Document 1)].

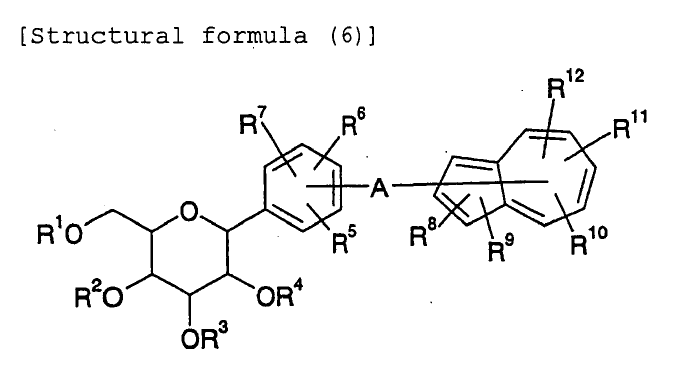

In the structural formula (6), individual symbols have the following meanings:
R₁ to R₄ are the same or different and are each hydrogen atom, lower alkyl, -C(=O)-lower alkyl, or -lower alkylene-aryl;
R₅ to R₁₂ are the same or different and are each hydrogen atom, lower alkyl, halogen-substituted lower alkyl, halogen atom, -OH, -O-lower alkyl, -lower alkylene-OH, -lower alkylene-O-lower alkyl, -O-lower alkylene-O-lower alkyl, -O-lower alkylene-aryl, -lower alkylene-O-C(=O)-lower alkyl, -COOH, nitro, amino, substituted amino, or -C(=O)-O-lower alkyl; and A is bond, lower alkylene or halogen-substituted lower alkylene,
wherein -A- may be bonded to any of the positions 1-8 of the azulene ring, and any two of R⁵, R⁶ and R⁷ may form a benzene ring together with the adjacent carbon atoms.

Of the azulene derivatives represented by the structural formula (6) described in the Patent Document 1, a process for producing (1S)-1,5-anhydro-1-C-[5-(azulen-2-ylmethyl)-2-hydroxyphenyl]-D-glucitol [hereinafter also referred to "compound (1)"] is found in the following reaction scheme (II), as seen in Reference Examples and Examples described in the Patent Document 1.

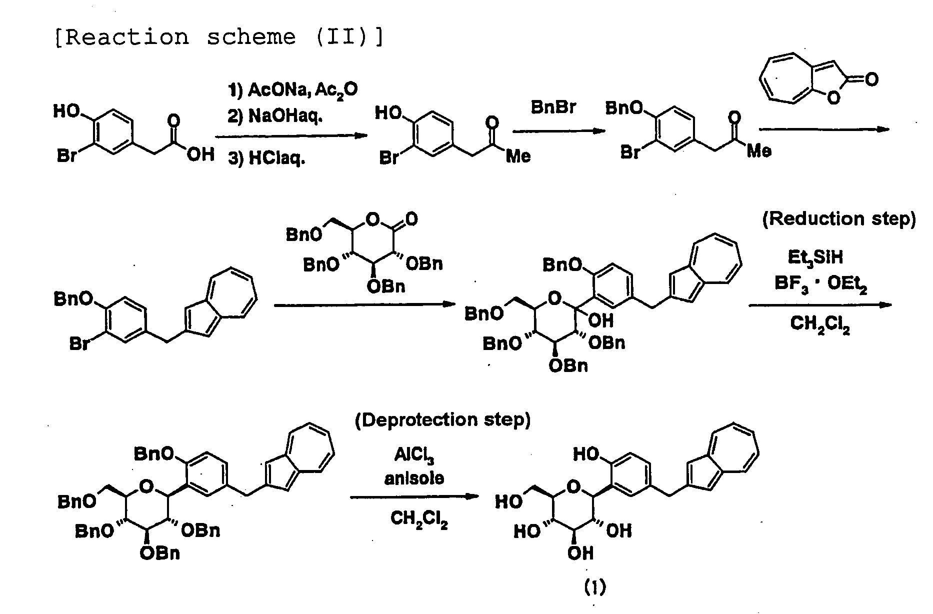

However, there were the following problems in industrial production of the compound (1) according to the above process of the Patent Document 1.

As shown in the Reference Examples 66, 67, 68 and 69 and Examples 74 and 75 of the Patent Document 1, the overall synthesis yield of the compound (1), as a target compound, is 1.4%, there has not been fully satisfactory industrially in yield and cost.

As shown in the Examples 74 and 75 of the Patent Document 1, methylene chloride is used as a reaction solvent in the reduction step and the deprotection step of the steps for obtaining the compound (1) as a target compound and, in the post-treatment, chloroform is used as a solvent for extraction and column chromatography. Use of these chlorine-containing solvents has not been fully satisfactory industrially from the standpoint of environmental protection.

As shown in the reference Examples 66, 67, 68 and 69 and Examples 74 and 75, purification operation by column chromatography is necessary in all the steps for obtaining the compound (1) as an intended compound, which has not been fully satisfactory industrially in yield, cost and environmental protection.

### Disclosure of the Invention

The present invention has been made in view of the above problems and aims at providing a process for producing an azulene derivative, which is high in yield, is simple in operation, is low in cost, is suited for environmental protection, and is advantageous industrially, as well as to a useful intermediate for synthesis of such an azulene derivative, obtained in the course of the process.

In order to achieve the above aim, the present inventors made intensive study on the process for industrial production of compound (1). As a result, it was found that, by using a particular intermediate, an azulene derivative can be produced without using any chlorine-containing solvent, without purification by column chromatography, in a high yield, in a simple operation, at a low cost, in a manner suited for environmental protection and with an industrial advantage to lead to the completion of the present invention. That is, the present invention provides the following process for producing an azulene derivative and the following intermediate for synthesis of the azulene derivative.

[1] A process for producing an azulene derivative, characterized by reducing and deprotecting at least one compound selected from a compound (3) represented by the following chemical formula (1): 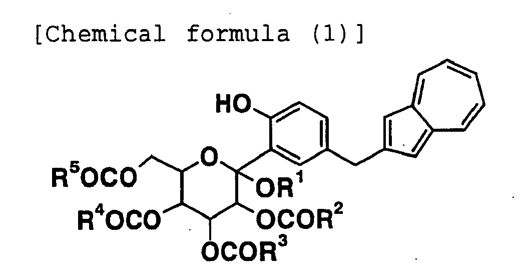 (wherein R¹ is a lower alkyl group, and R² to R⁵ may be the same or different and are each a lower alkyl group or an aryl group) or salt thereof, or at least one compound selected from a compound (2) which are each a mono-acylation product thereof, represented by the following chemical formula (2): 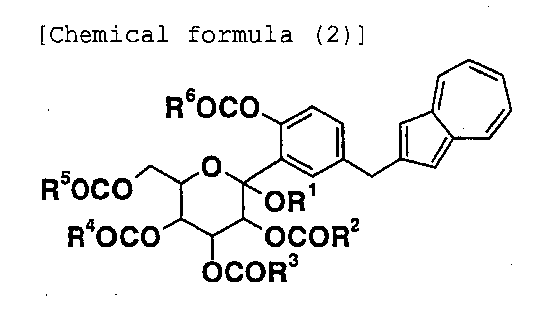 (wherein R¹ and R² to R⁵ have the same definitions as given above, and R⁵ may be the same as or different from R² to R⁵ and is a lower alkyl group or an aryl group), or a mixture of the compound (2) and the compound (3) or a mixture of a salt of the compound (2) and a salt of the compound (3), to obtain the following compound (1):

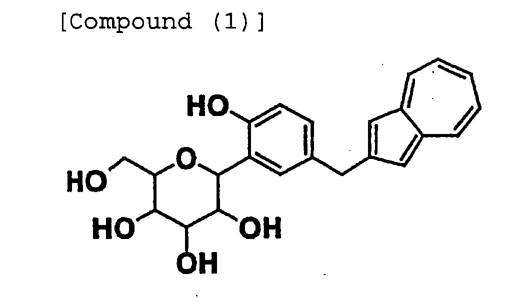

[2] The process for producing an azulene derivative according to [1], wherein the compound (3) represented by the chemical formula (1) contains methyl as R¹ and methyl as R² to R⁵ and is represented by the following structural formula (1) : 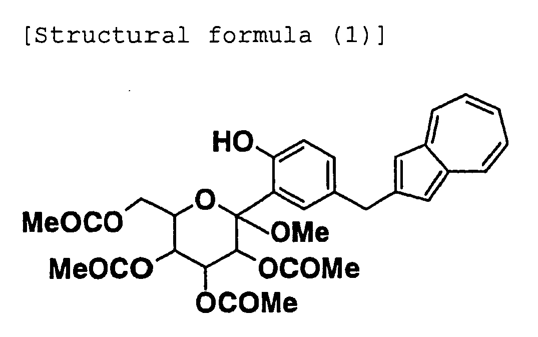 (wherein Me is methyl) .

[3] The process for producing an azulene derivative according to [1], wherein the compound (2) represented by the chemical formula (2) contains methyl as R¹ and methyl as R² to R⁵and R⁶ and is represented by the following structural formula (2) : 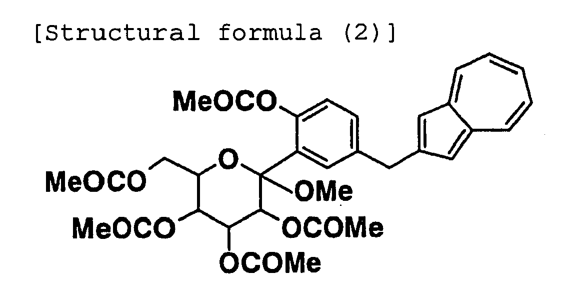 (wherein Me is methyl).

[4] A process for producing an azulene derivative, characterized by reducing and deprotecting at least one compound selected from a compound (3') represented by the following structural formula (1): 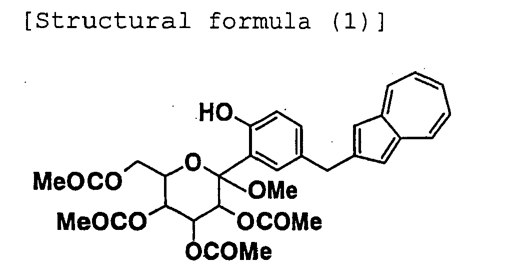 (wherein Me is methyl), or a compound (2') represented by the following structural formula (2): 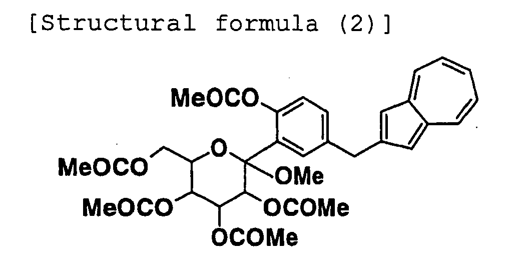 (wherein Me is methyl), or at least one compound selected from a salt of the compound (2') and a salt of the compound (3'), or a mixture of the compound (2') and the compound (3'), or a mixture of a salt of the compound (2') and a salt of the compound (3'), to obtain the following compound (1):

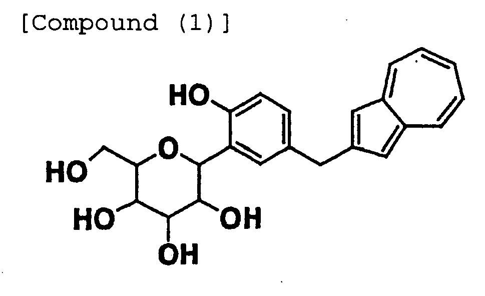

[5] The process for producing an azulene derivative according to any of [1] to [4], which comprises treating the following compound (4): 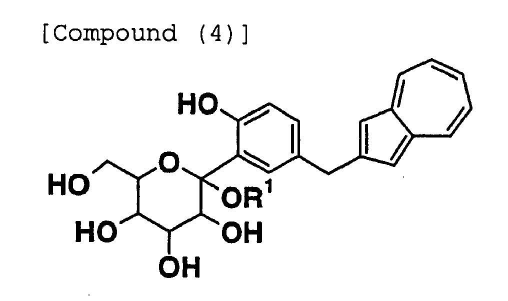 (wherein R¹ is a lower alkyl group) or salt thereof with an acylating agent to obtain the compound (3) represented by the chemical formula (1) and/or the compound (2) represented by the chemical formula (2).

[6] The process for producing an azulene derivative according to [5], wherein the compound (4) contains methyl as R¹ and is a compound represented by the following structural formula (3): 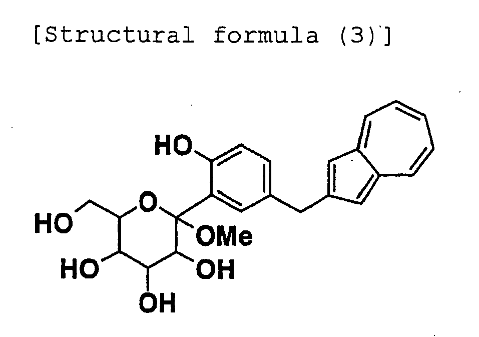 (wherein Me is methyl), and the acylating agent is an acetylating agent.

[7] The process for producing an azulene derivative, according to any of [1] to [6], wherein there is used, as the compound (4), a compound obtained by adding the following compound (5): 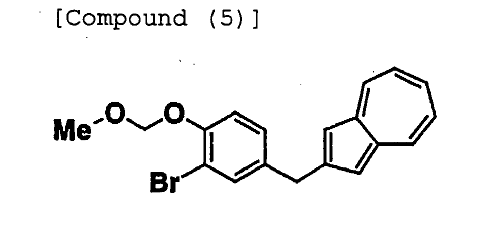 (wherein Me is methyl) to the following compound (6): 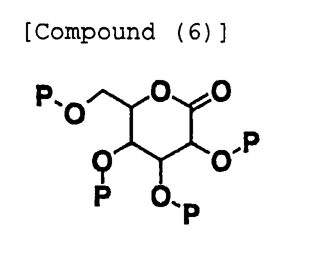 (wherein P is a trimethylsilyl group or a substitute protecting group thereof) to conduct deprotection and demethylglycosylation.

[8] The process for producing an azulene derivative, according to [7], wherein there is used, as the compound (5), a compound obtained by reacting the following compound (7): 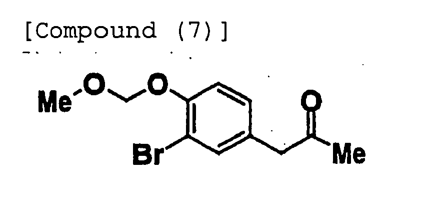 (wherein Me is methyl) with the following compound (8): 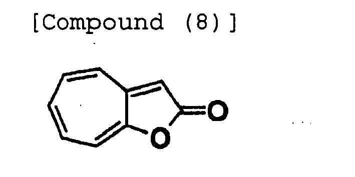 to form an azulene derivative.

[9] The process for producing an azulene derivative, set forth in [8], wherein there is used, as the compound (7), a compound obtained by methoxymethylating the following compound (9): 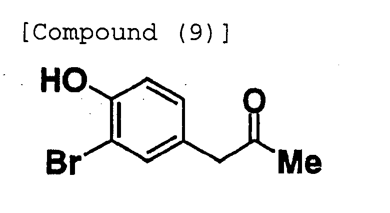 (wherein Me is methyl) or a salt thereof.

[10] A compound represented by the following general formula (1) : 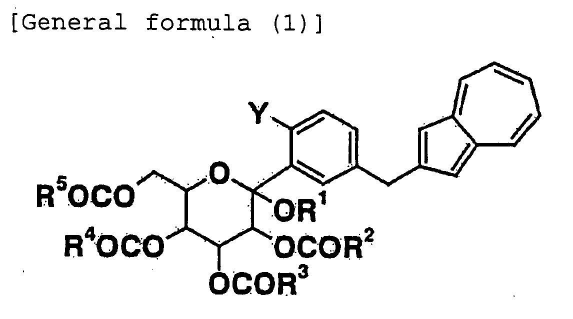 (wherein R¹ is a lower alkyl group, R² to R⁵ may be the same or different and are each a lower alkyl group or an aryl group, Y is -OH or -OCOR⁶, and R⁶ is a lower alkyl group or an aryl group) or a salt thereof.

[11] The compound of general formula (1) according to [10], wherein Y is -OCOR⁶ and R⁶ is a lower alkyl group or an aryl group, or a salt thereof.

[12] The compound of general formula (1) according to [11], which contains methyl as R¹ to R⁵. and methyl as R⁶ of -OCOR⁶ and is a compound represented by the following structural formula (2): 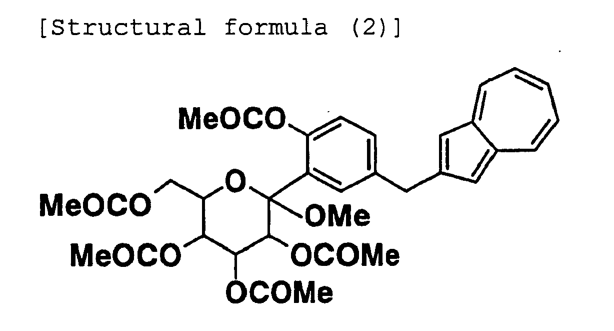 (wherein Me is methyl) .

[13] The compound of general formula (1) according to [9], wherein Y is -OH, or a salt thereof.

[14] The compound of general formula (1) according to [13], which contains methyl as R¹ to R⁵ and which is a compound represented by the following structural formula (1): 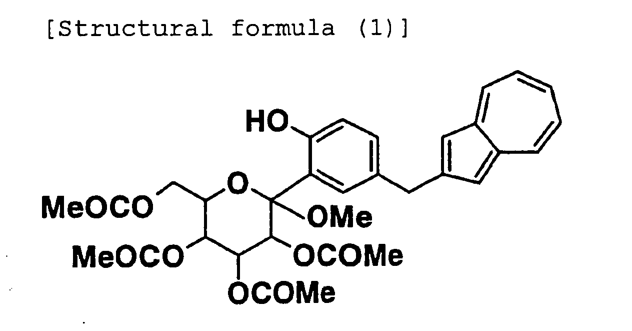 (wherein Me is methyl).

[15] A compound represented by the following structural formula (3): 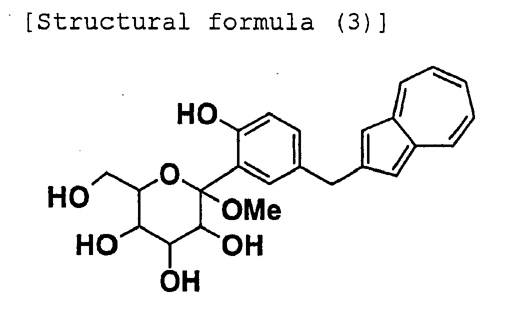 (wherein Me is methyl), or a salt thereof.

[16] A compound represented by the following structural formula (4): 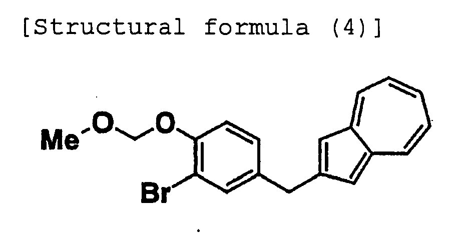 (wherein Me is methyl).

[17] A compound represented by the following structural formula (5): 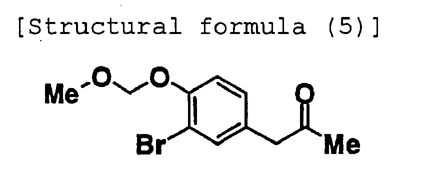 (wherein Me is methyl).

According to the present invention, there are provided a process for producing an azulene derivative, which is high in yield, is simple in operation, is low in cost, is suited for environmental protection, and is advantageous industrially, and an intermediate which is produced in the course of the above process and which is essential in the above process.

### Best Mode for Carrying Out the Invention

The best mode for carrying out the present invention is described specifically below.

Preferred steps (first step to fifth step) in the process of the present invention for production of azulene derivative are shown in the following reaction scheme (I), and the individual steps are specifically described below in the order of the fifth step to the first step.

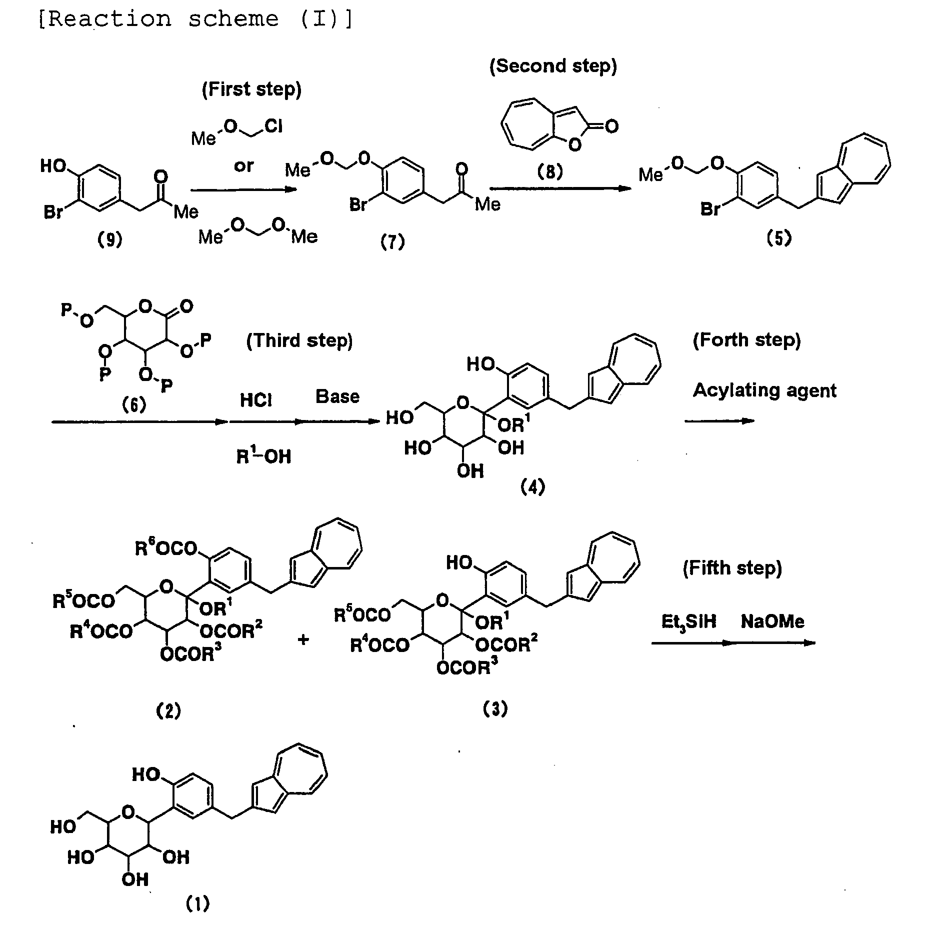

Before specifically describing the production process, description is made on a compound (hereinafter also referred to as "compound (2,3)") represented by the chemical formula (1) and/or the chemical formula (2). In the above reaction scheme (I), a compound represented by the chemical formula (1) is shown as compound (3) and a compound represented by the chemical formula (2) is shown as compound (2). Incidentally, the compound (3) represented by the following chemical formula (1): 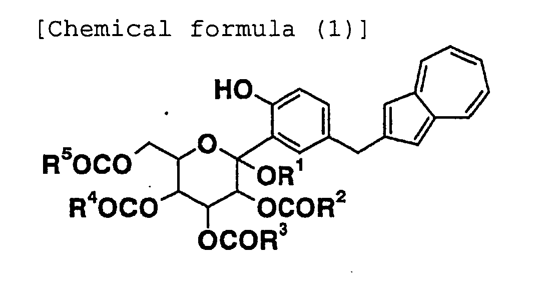 (wherein R¹ is a lower alkyl group and R² to R⁵ may be the same or different and are each a lower alkyl group or an aryl group) and the compound (2) which is a mono-acylation product thereof and is represented by the following chemical formula (2) : 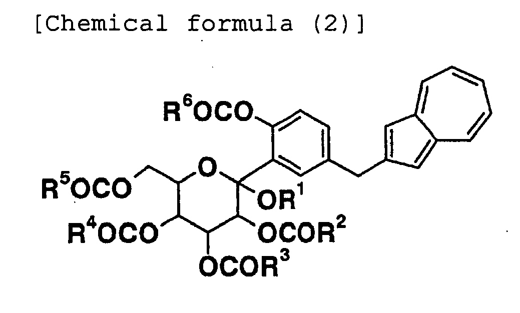 (wherein R¹ and R² to R⁵ have the same definitions as given above, and R⁶ may be the same as or different from R² to R⁵ and is a lower alkyl group or an aryl group) are each a novel substance. In the above, "lower alkyl" refers to an alkyl group of 1 to 6 carbon atoms, which may be branched; and "an aryl group" refers to, for example, a phenyl group which may be substituted. The lower alkyl group of the compound (4), represented by R¹ means as well a lower alkyl group of 1 to 6 carbon atoms, which may be branched. Incidentally, the compounds (2) and (3) as novel chemical substances are indicated by the general formula (1).

As a matter of course, a compound of the general formula (1) wherein R¹ and R² to R⁵ are each methyl and Y is -OH, corresponds to a compound (3') represented by the following structural formula (1): 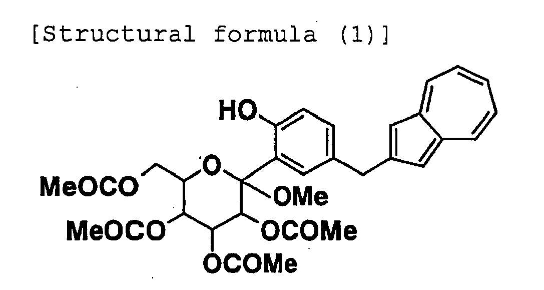 (wherein Me is methyl); and a compound of the general formula (1) wherein R¹ and R² to R⁵ are each methyl, Y is -OCOR⁶, and R⁶ is methyl, corresponds to a compound (2') represented by the following structural formula (2): 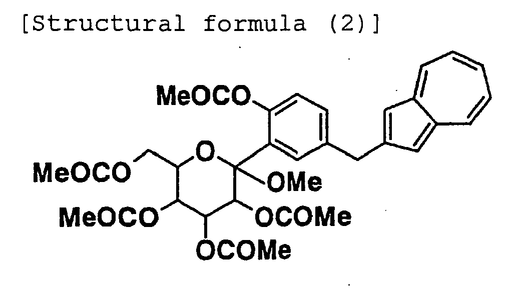 (wherein Me is methyl). The compound (3') and the compound (2') are each a novel substance and are useful as an intermediate for synthesis of the azulene derivative represented by the structural formula (6).

Incidentally, the compound represented by the structural formula (3), the compound (4) represented by the following formula: 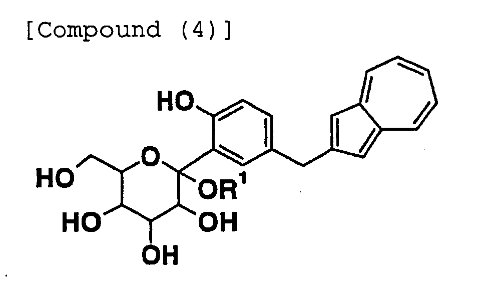 (wherein R¹ is a lower alkyl group), the compound represented by the structural formula (4), and the compound represented by the structural formula (5) are each a novel substance and are extremely useful as an intermediate for synthesis of the azulene derivative represented by the structural formula (6).

### (Fifth step)

The fifth step shown in the reaction scheme (I) is a step which comprises reducing and deprotecting the compound (2) and the compound (3), a salt of the compound (2), a salt of the compound (3), or any of the compound (2), a salt thereof, the compound (3) and a salt thereof to obtain the compound (1) [hereinafter also referred to as "compound (2, 3)"].

The reduction reaction is conducted in the presence of an appropriate reducing agent and an appropriate acid catalyst, in an appropriate solvent. As the reducing agent, there are mentioned triethylsilane, triisopropylsilane, tert-butyldimethylsilane, sodium borohydride, sodium triacetoxyborohydride, etc. Triethylsilane is preferred. As the acid, there are mentioned boron trifluoride-diethyl ether complex, trimethylsilyl trifluoromethanesulfonate, acetic acid, trifluoroacetic acid, etc. Trimethylsilyl trifluoromethanesulfonate is preferred. As the solvent, there are mentioned halogenated hydrocarbons such as methylene chloride, chloroform, 1,2-dichloroethane and the like; ethers such as diethyl ether, tetrahydrofuran and the like; acetonitrile; and so forth. Acetonitrile is preferred. The subsequent deprotection reaction is conducted in the presence of an appropriate base in an appropriate solvent.
As the base, there are mentioned metal hydroxides such as sodium hydroxide, potassium hydroxide and the like; metal alkoxides such as sodium methoxide, sodium ethoxide and the like; and so forth. Sodium methoxide is preferred. As the solvent, there are mentioned alcohols such as methanol, ethanol, isopropanol and the like; aromatic hydrocarbons such as benzene, toluene, xylene and the like; ethers such as diethyl ether, tetrahydrofuran and the like; water; and so forth. Methanol is preferred. The reduction reaction and the deprotection reaction are specifically conducted as follows. When there is used a compound (2,3), for example, a compound represented by the structural formula (1) or (2), at least one of these compounds is reacted in acetonitrile in the presence of an excess amount, preferably 4 equivalents of triethylsilane and an excess amount, preferably 3 equivalents of trimethylsilyl trifluoromethanesulfonate, under cooling or at room temperature, preferably at -10 to 0°C. This reaction is complete ordinarily in 5 to 20 hours. At this time, addition of an equivalent of water is preferred because the above reaction is accelerated. To the reaction mixture are added toluene and an aqueous sodium hydrogencarbonate solution to conduct extraction. The solvent in the organic layer is distilled off under reduced pressure. The residue is reacted in methanol in the presence of an excess amount, preferably 2 equivalents of sodium methoxide, under cooling or at room temperature, preferably at -5 to 5°C. This reaction is complete ordinarily in 1 to 2 hours. The reaction mixture is neutralized with an ethyl acetate solution of hydrogen chloride and then the solvent is distilled off under reduced pressure. To the residue are added ethyl acetate and water to conduct extraction. To the organic layer is added a diluted aqueous lithium hydroxide solution to conduct extraction. To the aqueous layer is added diluted hydrochloric acid to conduct neutralization, after which ethyl acetate is added to conduct extraction.
The solvent in the organic layer is distilled off under reduced pressure. To the residue are added isopropanol and water. The resulting crystals deposited are collected by filtration and dried, whereby a compound (1) can be obtained.

The compound (1) may also be obtained directly by subjecting a compound (4) to the above-mentioned reduction reaction. However, in this approach, the following compound (10) is produced as a by-product in a large amount; therefore, the above approach is not preferred as an industrial process. Meanwhile, the process of the present invention of producing a compound (1) by reducing and deprotecting a compound (2,3) which is obtained by acylation, for example, acetylation of a compound (4) as shown in the fourth step described later, is free from formation of the above-mentioned by-product and therefore is preferred as an industrial process. In addition, while the compound (2,3) including the compound represented by the structural formula (1) or (2), used in the present step can be easily produced also by a process known to those skilled in the art, a process shown in the following fourth step is advantageous as an industrial process.

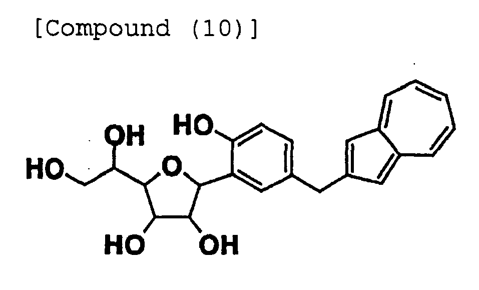

### (Fourth step)

The fourth step shown in the reaction scheme (I) is a step which comprises treating a compound (4) or a salt thereof, particularly a compound represented by the following structural formula (3): 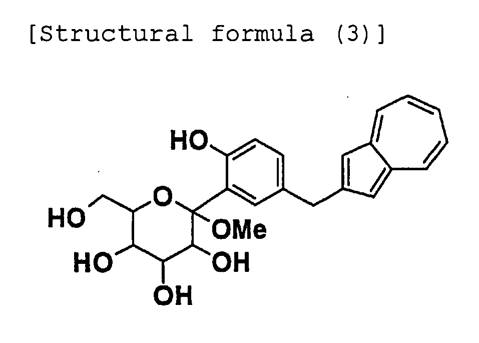 (wherein Me is methyl) or a salt thereof, with an acylating agent to obtain a compound (2,3).

The compound (2,3) can be produced by reacting a compound (4) or a salt thereof, particularly a compound represented by the structural formula (3) or a salt thereof with an acylating agent, for example, an acetylating agent in the presence of an appropriate base in an appropriate solvent. As the compound (4) used in this step, particularly the compound represented by the structural formula (3) (which is a compound (4) containing methyl as R¹) or a salt thereof, there is preferred the following sodium salt [a compound (4')].

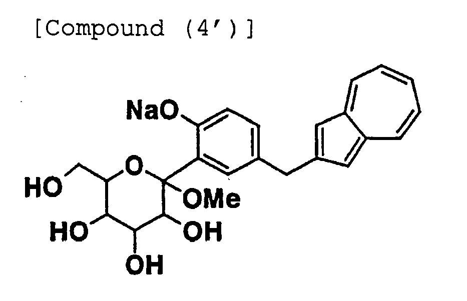

(wherein Me is methyl.)

As the solvent, there are mentioned ketones such as acetone, 2-butanone and the like; aromatic hydrocarbons such as benzene, toluene, xylene and the like; esters such as ethyl acetate, isopropyl acetate and the like; ethers such as diethyl ether, tetrahydrofuran and the like; aprotic polar solvents such as dimethylformamide, dimethylacetamide and the like; halogenated hydrocarbons such as methylene chloride, chloroform and the like; pyridine; water; and so forth.
Ethyl acetate is preferred. As the base, there are mentioned metal hydroxides such as sodium hydroxide, potassium hydroxide and the like; metal carbonates such as sodium carbonate, potassium carbonate and the like; metal alkoxides such as sodium methoxide, sodium ethoxide, potassium tert-butoxide and the like; metal hydrides such as sodium hydride and the like; tertiary amines such as triethylamine, diisopropylethylamine and the like; pyridine derivatives such as pyridine, lutidine and the like; and so forth. Pyridine is preferred.

As the acylating agent, there are mentioned halides of lower fatty acids, represented by acetyl chloride, propionyl chloride, acetyl bromide and the like; halides of aromatic carboxylic acids, such as benzoyl chloride and the like; anhydrides such as acetic anhydride, propionic anhydride, butyric anhydride and the like; and so forth. Of these, preferred are acetyl chloride, acetic anhydride, etc. and more preferred is acetic anhydride. The reaction is conducted, for example, by treating a compound (4) with an excess amount, preferably 6 equivalents of an acylating agent (e.g. acetic anhydride) in the presence of an excess amount, preferably 8 equivalents of pyridine in ethyl acetate under cooling or at room temperature; and it is complete ordinarily in 5 to 20 hours. In the reaction, addition of a catalytic amount of 4-dimethylaminopyridine is preferred because it accelerates the reaction. To the reaction mixture is added water or diluted hydrochloric acid to conduct extraction.
The organic layer is washed with an aqueous sodium hydrogencarbonate solution and brine and then the solvent is distilled off under reduced pressure, whereby a compound (2,3) including a compound represented by the structural formula (1) or (2) can be obtained at a high purity. Particularly when an acetylating agent is used as the acylating agent, a compound represented by the structural formula (1) or (2) can be obtained at a high purity.

The compound (3) obtained by the above reaction has a free form; however, it is possible to obtain the compound (3) as a corresponding salt or as a hydrate of free form or salt, when necessary. As the salt, there are mentioned, for example, inorganic (e.g. lithium, potassium, magnesium, calcium, sodium or aluminum) salts; organic amine (e.g. methylamine, ethylamine or ethanolamine) salts; basic amino acid (e.g. lysine or ornithine) salts; and an ammonium salt, and the like.

The compound (3) which is an intermediate in the synthesis of the present invention, includes a free form, a salt thereof, and their hydrates and polymorphism compounds. The compound (3) is preferably a free form when used as a raw material in the fifth step.

The compound (4) used in the present step, exemplified by the compound represented by the structural formula (3) can also be produced by a process known to those skilled in the art. However, the process shown in the third step described next is advantageous as a process for industrial production of the compound (4). That is, there is preferred a process which comprises adding a compound (5) to a compound (6) and conducting deprotection and methylglycosylation.

### (Third step)

The third step shown in the reaction scheme (I) is a step which comprises adding a compound (5) to a compound (6) in the presence of an alkyllithium reagent in an appropriate solvent, then treating the addition the resulting substance with an acid in the presence of methanol and, as necessary, treating the resulting substance with an appropriate base to produce a compound (4) including a compound represented by the structural formula (3), or a salt thereof.

As the salt of the compound (4) including the compound represented by the structural formula (3), there are mentioned, for example, inorganic (e.g. sodium, lithium, potassium, magnesium, calcium or aluminum) salts; organic amine (e.g. methylamine, ethylamine or ethanolamine) salts; basic amino acid (e.g. lysine or ornithine) salts; and an ammonium salt, and the like.

The compound (4) which is an intermediate in the synthesis of the present invention and which includes the compound represented by the structural formula (3), includes hydrates and polymorphism compounds. The compound (4) is preferably a sodium salt [compound (4')] or a free form when used as a raw material in the fourth step.

In the addition reaction, there are mentioned, as the alkyllithium reagent, n-butyllithium, sec-butyllithium, tert-butyllithium, etc. n-butyllithium is preferred. As the solvent, there are mentioned ethers such as diethyl ether, tetrahydrofuran and the like; aromatic hydrocarbons such as benzene, toluene, xylene and the like; and so forth. A mixed solvent of toluene and tetrahydrofuran is preferred. The reaction can be conducted by adding 0.95 to 1.05 equivalents, preferably 1.0 equivalent of n-butyllithium to a toluene-tetrahydrofuran (5:1) solution of a compound (5) at -80 to -60°C and adding the resulting reaction mixture to a toluene solution of 1.05 to 1.2 equivalents, preferably 1.2 equivalents of a compound (6) at -80 to -60°C. The reaction is complete at -80 to -60°C ordinarily in 2 to 24 hours.

In the subsequent step of treating the reaction mixture with an acid in the presence of methanol and then treating with an appropriate base, there are mentioned, as the acid, hydrogen chloride, sulfuric acid, acetic acid, trifluoroacetic acid, methanesulfonic acid, p-toluenesulfonic acid, etc. Hydrogen chloride is preferred. As the base, there are mentioned sodium hydroxide, sodium carbonate, and sodium alkoxides such as sodium methoxide, sodium ethoxide, and the like. Sodium hydroxide is preferred. The reaction can be conducted by adding the addition reaction mixture to an ethyl acetate-methanol solution of 3 equivalents of hydrogen chloride and reacting at -5 to 5°C ordinarily for 2 hours. The reaction mixture obtained is dispersed in an aqueous sodium hydroxide solution and the resulting crystals deposited are collected by filtration and dried, whereby can be obtained a compound (4) exemplified by a compound represented by the structural formula (3).

The protecting group of the compound (6) may be any protecting group as long as it is a substituent group bonded to oxygen, which can be converted to hydroxyl group without giving any harmful influence on other functional groups, in the third step. Specifically, there can be mentioned groups described in "Protective Groups in Organic Synthesis (third edition)" by Greene and Wuts. Of them, trimethylsilyl group is preferred.

The compound (5) used in the present step can be produced also by a process known to those skilled in the art. However, the process shown in the second step described next is advantageous as a process for industrial production.

### (Second step)

The second step shown in the reaction scheme (I) is a step which comprises reacting a compound (7) with an appropriate amine in an appropriate solvent and then reacting the reaction product with a compound (8) to produce a compound (5).

In the reaction of the compound (7) with an amine, there are mentioned, as the amine, morpholine, pyrrolidine, N-methylpiperazine, diethylamine, diisopropylamine, etc. Pyrrolidine is preferred. As the solvent, there are mentioned aromatic hydrocarbons such as benzene, toluene, xylene and the like; ethers such as diethyl ether, tetrahydrofuran and the like; alcohols such as methanol, ethanol, isopropanol and the like; halogenated hydrocarbons such as methylene chloride, chloroform and the like; and so forth. Toluene is preferred. A dehydrating agent such as magnesium sulfate, sodium sulfate, molecular sieve or the like may be added. In industrial production, there is preferred, for simplicity of operation, a method of azeotropic distillation of solvent and water to remove the water generated in the reaction. Specifically, the method is conducted while heating the compound (7) with 1 to 3 equivalents of pyrrolidine in toluene to 40°C to the reflux temperature and distilling off the solvent under reduced pressure or at normal pressure. When the reaction has stopped, pyrrolidine is supplemented appropriately, whereby the reaction can be completed.

In the subsequent reaction with a compound (8), there are mentioned, as the solvent, aromatic hydrocarbons such as benzene, toluene, xylene and the like; ethers such as diethyl ether, tetrahydrofuran and the like; alcohols such as methanol, ethanol, isopropanol and the like; halogenated hydrocarbons such as methylene chloride, chloroform and the like; acetonitrile; dimethylformamide; dimethyl sulfoxide; and so forth. The reaction is preferably conducted in isopropanol under heating and refluxing. The reaction is complete ordinarily in 5 to 25 hours. The solvent in the reaction mixture is distilled off under reduced pressure. Toluene and diluted hydrochloric acid are added to the residue to conduct extraction. At this time, insolubles separate out; therefore, it is appropriate that the insolubles are removed and then phase separation is made.
The organic layer is washed with an aqueous sodium hydrogencarbonate solution and brine and then the solvent is distilled off under reduced pressure. Isopropanol and heptane are added to the residue. The resulting crystals deposited are collected by filtration and dried, whereby a compound (5) can be obtained at a high purity.

In order to obtain a compound (5) from a compound (7) by using a substance other than amine, it is possible to convert a compound (7) to corresponding enol ether and reacting it with a compound (8).

The compound (7) used in the present step can be produced also by a process known to those skilled in the art. However, the process shown in the first step described next is advantageous as a process for industrial production.

### (First step)

The first step shown in the reaction scheme (I) is a step which comprises protecting the phenolic hydroxyl group of a compound (9) to obtain a compound (7). There is shown below a process of introducing methoxymethyl which is a preferred protecting group. The compound (7) can be produced by allowing chloromethyl methyl ether to act on a compound (9) in the presence of a base in an appropriate solvent. As the solvent, there are mentioned ketones such as acetone, 2-butanone and the like; aromatic hydrocarbons such as benzene, toluene, xylene and the like; acetic acid esters such as ethyl acetate, isopropyl acetate and the like; ethers such as diethyl ether, tetrahydrofuran and the like; aprotic polar solvents such as dimethylformamide, dimethylacetamide and the like; halogenated hydrocarbons such as methylene chloride, chloroform and the like; and so forth. Acetone is preferred. As the base, there are mentioned metal hydrides such as sodium hydride and the like; tertiary amines such as triethylamine, diisopropylethylamine and the like; metal carbonates such as potassium carbonate and the like; and so forth. Potassium carbonate is preferred. The reaction is conducted by allowing 1.1 to 2 equivalents, preferably 1.5 equivalents of potassium carbonate and 1.1 to 1.5 equivalents, preferably 1.5 equivalents of chloromethyl methyl ether to act on a compound (9) in acetone. The reaction can be conducted under cooling or at room temperature, but is preferably conducted at -5 to 5°C ordinarily for 30 minutes to 1 hour. The resulting reaction mixture are added water and toluene to conduct extraction. The organic layer is washed with a diluted aqueous sodium hydroxide solution to remove unreacted compound (9) and then the solvent is distilled off under reduced pressure, whereby a compound (7) can be obtained at a high purity.

Meanwhile, it is desired to avoid using chloromethyl methyl ether in mass synthesis, in view of the toxicity. Hence, a compound (7) can also be produced from a compound (9) by using methylal as a substitute reagent for chloromethyl methyl ether. That is, methylal is allowed to act on a compound (9) in the presence of an acid or a dehydrating agent in an appropriate solvent. As the solvent, there are mentioned ketones such as acetone, 2-butanone and the like; aromatic hydrocarbons such as benzene, toluene, xylene and the like; acetic acid esters such as ethyl acetate, isopropyl acetate and the like; ethers such as diethyl ether, tetrahydrofuran and the like; aprotic polar solvents such as dimethylformamide, dimethylacetamide and the like; halogenated hydrocarbons such as methylene chloride, chloroform and the like; and so forth. Methylal may be used as the solvent; however, toluene is preferred as the solvent. As the acid, there are mentioned Brønsted acids such as sulfuric acid, methanesulfonic acid, p-toluenesulfonic acid, acetic acid, trifluoroacetic acid and the like; Lewis acids such as aluminum chloride, iron chloride, trimethylsilyl trifluoromethanesulfonate and the like; and so forth. As the dehydrating agent, there are mentioned diphosphorus pentoxide, molecular sieve, etc. Diphosphorus pentoxide is preferred. The reaction is conducted by allowing 3 to 4 equivalents, preferably 3 equivalents of diphosphorus pentoxide and 8 to 25 equivalents, preferably 10 equivalents of methylal to act on a compound (9) in toluene. The reaction can be conducted under cooling or at room temperature, but is preferably conducted at -5 to 5°C ordinarily for 3 to 19 hours. To the resulting reaction mixture are added water and toluene to conduct extraction. The organic layer is washed with an aqueous potassium carbonate solution to remove unreacted compound (9) and then the solvent is distilled off under reduced pressure, whereby a compound (7) can be obtained at a high purity.

As the protecting group other than methoxymethyl, any group can be used as long as it is a substituent group bonded to oxygen mentioned in the third step, which can be converted to hydroxyl group without giving any harmful influence on other functional groups in the second and third steps. Specifically, there can be mentioned groups described in the above-mentioned "Protective Groups in Organic Synthesis (third edition)".

Introduction of a protecting group other than methoxymethyl can be conducted by a process known to those skilled in the art, as well. It can be conducted, for example, based on the process described in the above-mentioned "Protective Groups in Organic Synthesis (third edition)".

In the production process of the present invention, appropriate intermediates are used and, as a protective group for hydroxyl group, there are used methoxymethyl group, trimethylsilyl group and acetyl group without benzyl group; thereby, deprotection has been made easy under a mild acidic or basic condition and a significant improvement in yield has been achieved. Further, chlorine-containing solvent is avoided to use in all the steps and no purification operation by column chromatography is conducted; thereby, obtainment of a compound (1) at a high purity has been made possible. Thus, the production process of the present invention is high in yield, simple in operation, low in cost, suited for environmental protection, and advantageous industrially, and is highly useful.

### Examples

The present invention is described more specifically below by way of Examples. However, the present invention is in no way restricted by these Examples. The synthesis processes of compound (1) (Reference Examples 1 to 6) described in the Patent Document 1 and the synthesis process (Reference Example 7) of a raw material compound are shown below as Reference Examples 1 to 7.

### (Reference Example 1)

### Synthesis of 1-(3-bromo-4-hydroxyphenyl)acetone

Sodium acetate (50.5 g) was added to an acetic anhydride (100 ml) solution of 3-bromo-4-hydroxyphenylacetic acid (28.5 g). The mixture was refluxed for 21 hours with heating. The reaction mixture was returned to room temperature and was adjusted to pH 11 by adding a 20% aqueous sodium hydroxide solution, and then the mixture was refluxed for 1 hour with heating. The reaction mixture was returned to room temperature and was adjusted to pH 6 by adding a 10% aqueous hydrochloric acid solution. The whole was extracted with ethyl acetate. The organic layer was washed with water, a saturated aqueous sodium bicarbonate solution and saturated brine, and dried over anhydrous sodium sulfate. After filtration, the solvent was distilled off under reduced pressure. The residue was purified by silica gel column chromatography (ethyl acetate-n-hexane) to obtain 1-(3-bromo-4-hydroxyphenyl)acetone (22.2 g, yield = 79%).

### (Reference Example 2)

### Synthesis of 1-[4-(benzyloxy)-3-bromophenyl]acetone

To a solution of 1-(3-bromo-4-hydroxyphenyl)acetone (4.0 g) in DMF (40 ml) were added potassium carbonate (2.7 g) and benzyl bromide (2.3 ml). The mixture was stirred for 6 hours at room temperature. The reaction mixture was poured into water, and the whole was extracted with ethyl acetate. The organic layer was washed with water and saturated brine and then dried over anhydrous sodium sulfate. After filtration, the solvent was distilled off under reduced pressure. The resulting residue was purified by silica gel column chromatography (ethyl acetate-n-hexane) to obtain 1-[4-(benzyloxy)-3-bromophenyl]acetone (3.65 g, yield = 66%).

### (Reference Example 3)

### Synthesis of 2-[4-(benzyloxy)-3-bromobenzyl]azulene

Pyrrolidine (1.9 ml) and magnesium sulfate (2.74 g) were added to a solution of 1-[4-(benzyloxy)-3-bromophenyl]acetone (3.65 g) in diethyl ether (30 ml). The mixture was stirred for 12 hours at room temperature. After filtration, the solvent was distilled off under reduced pressure and the residue was dried under reduced pressure. The resulting residue was dissolved in ethanol (30 ml) and 2H-cyclohepta[b]furan-2-one (0.5 g) was added to the solution. The mixture was refluxed for 8 hours with heating. The reaction mixture was concentrated and the resulting residue was purified by silica gel column chromatography (ethyl acetate-n-hexane) to obtain 2-[4-(benzyloxy)-3-bromobenzyl]azulene (0.84 g, yield = 61%).

### (Reference Example 4)

### Synthesis of 1-C-[5-(azulen-2-ylmethyl)-2-(benzyloxy)phenyl]-2,3,4,6-tetra-o-benzyl-D-glucopyranose

To a solution of 2-[4-(benzyloxy)-3-bromobenzyl]azulene (0.25 g, 0.62 mmol) in THF (4.0 ml) was dropwise added, at -50°C, a 1.58 M n-butyllithium n-hexane solution (0.39 ml, 0.62 mmol). The mixture was stirred for 30 minutes at the same temperature. To the solution was dropwise added a solution of 2,3,4,6-tetra-o-benzyl-glucono-1,5-lactone (0.28 g, 0.52 mmol) in THF (4.0 ml). The mixture was stirred for 30 minutes at the same temperature and further for 30 minutes at 0°C. Water was added to the reaction mixture and the whole was extracted with ethyl acetate. The organic layer was washed with brine and dried over anhydrous sodium sulfate. After filtration, the solvent was distilled off under reduced pressure. The resulting residue was purified by silica gel column chromatography [ethyl acetate-n-hexane (1:4)] to obtain 1-C-[5-(azulen-2-ylmethyl)-2-(benzyloxy)phenyl]-2,3,4,6-tetra-o-benzyl-D-glucopyranose (0.16 g, 0.20 mmol, yield = 38%).

### (Reference Example 5)

### Synthesis of (1S)-1,5-anhydro-1-[5-(azulen-2-ylmethyl)-2-(benzyloxy)phenyl]-2,3,4,6-tetra-O-benzyl-D-glucitol

To a solution of 1-C-[5-(azulen-2-ylmethyl)-2-(benzyloxy)phenyl]-2,3,4,6-tetra-o-benzyl-D-glucopyranose (1.80 g, 2.09 mmol) in acetonitrile (20 ml) were added triethylsilane (1.0 ml, 6.26 mmol) and trifluoroacetic acid (0.33 ml, 4.28 mmol). The mixture was stirred for 30 minutes at -15°C and further for 14 hours at room temperature. Triethylsilane (1.0 ml, 6.26 mmol) and trifluoroacetic acid (0.33 ml, 4.28 mmol) were further added to the reaction mixture and stirred for 3 hours at room temperature. The reaction mixture was poured into a saturated aqueous sodium hydrogencarbonate solution and the whole was extracted with ethyl acetate. The organic layer was washed with saturated brine and dried over anhydrous sodium sulfate. After filtration, the solvent was distilled off under reduced pressure. The resulting residue was purified by silica gel column chromatography [ethyl acetate-n-hexane (1:4)] to obtain (1S)-1,5-anhydro-1-[5-(azulen-2-ylmethyl)-2-(benzyloxy)phenyl]-2,3,4,6-tetra-0-benzyl-D-glucitol (0.65 g, 0.77 mmol, yield = 37%).

### (Reference Example 6)

### Synthesis of (1S)-1,5-anhydro-1-[5-(azulen-2-ylmethyl)-2-hydroxyphenyl]-D-glucitol

To a solution of (1S)-1,5-anhydro-1-[5-(azulen-2-ylmethyl)-2-(benzyloxy)phenyl]-2,3,4,6-tetra-O-benzyl-D-glucitol (0.07 g) in methylene chloride (5 ml) were added aluminum chloride (0.12 g) and anisole (4.0 ml). The mixture was stirred for 2 hours at room temperature. The reaction mixture was poured into ice water and the whole was extracted with ethyl acetate. The organic layer was washed with brine and dried over anhydrous sodium sulfate. After filtration, the filtrate was concentrated. The resulting residue was purified by silica gel column chromatography (chloroformmethanol) to obtain (1S)-1,5-anhydro-1-[5-(azulen-2-ylmethyl)-2-hydroxyphenyl]-D-glucitol (0.01 g, yield = 31%).

### (Reference Example 7)

### Synthesis of 1-(3-bromo-4-hydroxyphenyl)acetone

To a solution of 1-(4-hydroxyphenyl)acetone (50 g, 333 mmol) in methanol (250 ml) was added 1, 3-dibromo-5, 5-dimethylhydantoin (49.5 g) at -10 to 0°C. The mixture was stirred for 30 minutes at 0 to 5°C. The solvent was distilled off under reduced pressure. To the residue were added ethyl acetate (300 ml), toluene (100 ml) and water (400 ml) to conduct extraction. The organic layer was washed with water (400 ml) and the solvent was distilled off under reduced pressure. To the resulting residue was added toluene (150 ml), and the mixture was cooled to 0°C and seeded crystals and stirred for 16 hours. The resulting crystals deposited were collected by filtration, washed with cooled toluene (50 ml), and dried under reduced pressure to obtain 1-(3-bromo-4-hydroxyphenyl)acetone [58.32 g, yield = 77%, purity = 96% (HPLC)] as white crystals.

### (Example 1)

### Synthesis of 1-[3-bromo-4-(methoxymethoxy)phenyl]acetone

To a solution of 1-(3-bromo-4-hydroxyphenyl)acetone (515.05 g, 2.25 mol) in acetone (2.58 liters) were added potassium carbonate (466.61 g) and chloromethyl methyl ether (272.23 g). The mixture was stirred for 30 minutes at -5 to 5°C. The reaction mixture was extracted by adding toluene (4.2 liters) and water (4.2 liters). The organic layer was washed with a 0.1 M aqueous sodium hydroxide solution (4.2 liters) and subsequently twice with water (4.2 liters). The solvent was distilled off under reduced pressure to obtain 1-[3-bromo-4-(methoxymethoxy)phenyl]acetone [605.25 g, yield = 99%, purity = 96% (HPLC)] as a light yellow oily substance.

### (Example 2)

### Synthesis of 1-[3-bromo-4-(methoxymethoxy)phenyl]acetone

To a solution of diphosphorus pentoxide (1.70 g) in toluene (9 ml) were added methylal (3.04 g) and 1-(3-bromo-4-hydroxyphenyl)acetone (0.915 g, 3.99 mmol). The mixture was stirred for 17 hours at -5 to 5°C. The reaction mixture was extracted by adding water (9 ml). The organic layer was washed twice with a 10% aqueous potassium carbonate solution (9 ml) and then the solvent was distilled off under reduced pressure to obtain 1-[3-bromo-4-(methoxymethoxy)phenyl]acetone [1.09 g, yield = 100%, purity = 94% (HPLC)] as a light yellow oily substance.

### (Example 3)

### Synthesis of 2-[3-bromo-4-(methoxymethoxy)benzyl]azulene

Pyrrolidine (41 ml) was added to a solution of 1-[3-bromo-4-(methoxymethoxy)phenyl]acetone (68.7 g, 251.54 mmol) in toluene (690 ml). The mixture was subjected to atmospheric distillation to distill off the solvent by 73 ml. Pyrrolidine (20 ml) was added and the mixture was subjected to atmospheric distillation to distill off the solvent by 27 ml. The reaction mixture was cooled and the solvent was distilled off under reduced pressure. 2H-cyclohepta[b]furan-2-one (40 g) and isopropanol (370 ml) were added to the resulting residue, the mixture was refluxed for 19 hours with heating. The reaction mixture was concentrated. To the residue were added toluene (690 ml) and 1 M hydrochloric acid (690 ml). After decantation of the supernatant liquid to remove the resulting insolubles deposited, the aqueous and organic layers were separated. To the organic layer was again added 1 M hydrochloric acid (690 ml). After decantation of the supernatant liquid to remove the resulting insolubles deposited, the aqueous and organic layers were separated. This operation was conducted one more time. The organic layer was washed with a 5% aqueous sodium hydrogencarbonate solution (690 ml) and 25% brine (690 ml) and then the solvent was distilled off under reduced pressure. Isopropanol (280 ml) and n-heptane (70 ml) were added to the resulting residue. The resulting crystals deposited after seeding at 15°C were collected by filtration, then washed with cooled isopropanol (35 ml), and dried under reduced pressure to obtain 2-[3-bromo-4-(methoxymethoxy)benzyl]azulene [25.32 g, yield = 26%, purity = 97% (HPLC)] as blue crystals.

### (Example 4)

### Synthesis of sodium 4-(azulen-2-ylmethyl)-2-(1-methoxy-D-glucopyranosyl)phenolate

To a solution of 2-[3-bromo-4-(methoxymethoxy)benzyl]azulene (5.00 g, 14.00 mmol) in toluene (84 ml)-THF (17 ml) was dropwise added, at -75 to -70°C, a 1.58 M n-butyllithium n-hexane solution (8.9 ml). The mixture was stirred for 30 minutes at the same temperature. The reaction mixture was added, at -75 to -65°C, to a solution of 2,3,4,6-tetrakis-O-(trimethylsilyl)-D-glucono-1,5-lactone (7.85 g) in toluene (50 ml). The mixture was stirred for 19 hours at -60°C. The reaction mixture was added, at -10 to 0°C, to a solution of a 4 M hydrogen chloride ethyl acetate solution (10.9 g) in methanol (25 ml). The mixture was stirred for 3 hours at the same temperature. The reaction mixture was added, at 0 to 10°C, to a solution of sodium hydroxide (4.02 g) in water (100 ml). The mixture was stirred for 3 hours at the same temperature. The resulting crystals deposited were collected by filtration, washed with water (5 ml) and toluene (5 ml), and suspended in water (50 ml) and stirred for 15 hours at 0°C. After filtration, the resulting crystals were washed with water (5 ml) and then dried under reduced pressure to obtain sodium 4-(azulen-2-ylmethyl)-2-(1-methoxy-D-glucopyranosyl)phenolate [3.14 g, yield = 50%, purity = 90% (HPLC)] as blue crystals.

In addition, the name of the free form of the above-obtained salt is methyl 1-C-[5-(azulen-2-ylmethyl)-2-hydroxyphenyl]-D-glucopyranoside.

### (Example 5)

### Synthesis of methyl 1-C-[5-(azulen-2-ylmethyl)-2-hydroxyphenyl]-D-glucopyranoside

To a solution of 2-[3-bromo-4-(methoxymethoxy)benzyl]azulene (3.0 g, 8.39 mmol) in tetrahydrofuran (30 ml) was dropwise added, at -45°C in argon atmosphere, a 1.58 M n-butyllithium n-hexane solution (6.1 ml, 9.64 mmol). The mixture was stirred for 15 minutes at the same temperature. The resulting solution was dropwise added, at -45°C, to a solution of 2,3,4,6-tetrakis-O-(trimethylsilyl) -D-glucono-1, 5-lactone (4.7 g, 10.08 mmol) in toluene (30 ml). The mixture was stirred for 30 minutes at the same temperature. To the reaction mixture was added a solution obtained by adding, to methanol (15 ml), a 4 M hydrogen chloride ethyl acetate solution (6.3 ml, 25.17 mmol). The mixture was stirred for 2 hours at 0°C. To the reaction mixture was added a 1 M aqueous sodium hydroxide solution (50 ml), and the whole was extracted with toluene. The organic layer was extracted with a 1 M aqueous sodium hydroxide solution. To the combined aqueous layers was added 10% hydrochloric acid to adjust pH 5.5. Then, the mixture was extracted with ethyl acetate. The organic layer was dried over anhydrous magnesium sulfate and filtered. After filtration, the solvent was distilled off under reduced pressure. The resulting residue was crystallized from 2-propanol-water to obtain methyl 1-C-[5-(azulen-2-ylmethyl)-2-hydroxyphenyl]-D-glucopyranoside (1.2 g, 2.81 mmol, yield = 33%).

### (Example 6)

### Synthesis of a mixture of methyl 2,3,4,6-tetra-O-acetyl-1-C-[2-(acetyloxy)-5-(azulen-2-ylmethyl)phenyl]-D-glucopyranoside and methyl 2,3,4,6-tetra-O-acetyl-1-C-[5-(azulen-2-ylmethyl)-2-hydroxyphenyl]-D-glucopyranoside

To a solution of sodium 4-(azulen-2-ylmethyl)-2-(1-methoxy-D-glucopyranosyl)phenolate (3.00 g, 6.69 mmol)in ethyl acetate (30 ml) were added pyridine (4.23 g), 4-dimethylaminopyridine (8 mg) and acetic anhydride (4.10 g). The mixture was stirred for 17 hours at room temperature. Water (30 ml) was added to the reaction mixture to conduct extraction. The organic layer was washed with water (30 ml) three times, a 5% aqueous sodium hydrogencarbonate solution and 25% brine (30 ml) and then the solvent was distilled off under reduced pressure to obtain a mixture of methyl 2,3,4,6-tetra-O-acetyl-1-C-[2-(acetyloxy)-5-(azulen-2-ylmethyl)phenyl]-D-glucopyranoside and methyl 2,3,4,6-tetra-O-acetyl-1-C-[5-(azulen-2-ylmethyl)-2-hydroxyphenyl]-D-glucopyranoside as a blue amorphous substance. The mixture was 4.24 g and 100% in yield (calculated based on methyl 2,3,4,6-tetra-O-acetyl-1-C-[2-(acetyloxy)-5-(azulen-2-ylmethyl)phenyl]-D-glucopyranoside). The proportions on HPLC, of methyl 2,3,4,6-tetra-O-acetyl-1-C-[2-(acetyloxy)-5-(azulen-2-ylmethyl)phenyl]-D-glucopyranoside and methyl 2,3,4, 6-tetra-O-acetyl-1-C-[5- (azulen-2-ylmethyl) -2-hydroxyphenyl]-D-glucopyranoside were 40% and 42%, respectively.

### (Example 7)

### Synthesis of (1S)-1,5-anhydro-1-[5-(azulen-2-ylmethyl)-2-hydroxyphenyl]-D-glucitol

To a solution of methyl 2,3,4,6-tetra-O-acetyl-1-C-[2-(acetyloxy)-5-(azulen-2-ylmethyl)phenyl]-D-glucopyranoside (3.34 g, 5.25 mmol) in acetonitrile (33 ml) were added water (0.09 g), triethylsilane (2.44 g) and trimethylsilyl trifluoromethanesulfonate (3.56 g). The mixture was stirred for 15 hours at -5°C. Toluene (50 ml) and a 5% aqueous sodium hydrogencarbonate solution (50 ml) were added to the reaction mixture to conduct extraction. The solvent in the organic layer was distilled off under reduced pressure. Methanol (33 ml) and a 28% sodium methoxide methanol solution (2.02 g) were added to the resulting residue and the mixture was stirred for 2 hours at 0°C. To the reaction mixture was added a 4 M hydrogen chloride ethyl acetate solution (2.63 ml) for neutralization. The solvent was distilled off under reduced pressure. Ethyl acetate (67 ml) and water (67 ml) were added to the resulting residue to conduct extraction. The organic layer was washed with water (33 ml). The aqueous layer was extracted with ethyl acetate (67 ml). The organic layer was combined with the first organic layer, and the combined organic layer was extracted with a 0.5% aqueous lithium hydroxide solution (67 ml). The organic layer was extracted twice with a 0.5% aqueous lithium hydroxide solution (33 ml). The aqueous layer obtained was neutralized with 1 M hydrochloric acid (0.80 ml), followed by extraction with ethyl acetate (67 ml). The aqueous layer was extracted again with ethyl acetate (34 ml). The organic layers obtained were combined and the solvent was distilled off under reduced pressure. The resulting residue obtained was dissolved in isopropanol (1.8 ml) and water (1.8 ml) and then water (3.6 ml) was added. The mixture was stirred at room temperature. The resulting crystals deposited were collected by filtration, washed twice with an isopropanol-water (1:3) mixed solvent (1.8 ml) and dried under reduced pressure to obtain (1S)-1,5-anhydro-1-[5-(azulen-2-ylmethyl)-2-hydroxyphenyl]-D-glucitol [1.43 g, yield = 69%, purity 93% (HPLC)] as blue crystals.

### (Example 8)

### Synthesis of (1S)-1,5-anhydro-1-[5-(azulen-2-ylmethyl)-2-hydroxyphenyl]-D-glucitol

To a solution of methyl 2,3,4,6-tetra-O-acetyl-1-C-[5-(azulen-2-ylmethyl)-2-hydroxyphenyl]-D-glucopyranoside (4.60 g, 7.73 mmol) in acetonitrile (46 ml) were added water (0.14 g), triethylsilane (3.60 g) and trimethylsilyl trifluoromethanesulfonate (5.25 g). The mixture was stirred for 16 hours at -5°C. Toluene (69 ml) and a 5% aqueous sodium hydrogencarbonate solution (69 ml) were added to the reaction mixture to extract. The solvent in the organic layer was distilled off under reduced pressure. Methanol (46 ml) and a 28% sodium methoxide methanol solution (2.98 g) were added to the resulting residue, and the mixture was stirred for 2 hours at 0°C. To the reaction mixture was added a 4 M hydrogen chloride ethyl acetate solution (3.87 ml) for neutralization. The solvent was distilled off under reduced pressure. Ethyl acetate (92 ml) and water (92 ml) were added to the resulting residue to extract. The organic layer was washed with water (46 ml). The aqueous layer was extracted with ethyl acetate (92 ml). The organic layer was combined with the first organic layer, and the combined organic layer was extracted with a 0.5% aqueous lithium hydroxide solution (92 ml). The organic layer was extracted twice with a 0.5% aqueous lithium hydroxide solution (46 ml). The aqueous layer extracted was neutralized with 1 M hydrochloric acid (2.19 ml), followed by extraction with ethyl acetate (92 ml). The aqueous layer was extracted again with ethyl acetate (46 ml). The organic layers extracted were combined and the solvent was distilled off under reduced pressure. The residue obtained was dissolved in isopropanol (3.28 ml) and water (3.28 ml). Water (6.56 ml) was added thereto, followed by stirring at room temperature. The resulting crystals deposited were collected by filtration, washed four times with an isopropanol-water (1:3) mixed solvent (1.64 ml) and dried under reduced pressure to obtain (1S)-1,5-anhydro-1-[5-(azulen-2-ylmethyl)-2-hydroxyphenyl]-D-glucitol [2.12 g, yield = 69%, purity 94% (HPLC)] as blue crystals.

### (Example 9)

### Synthesis of (1S)-1,5-anhydro-1-[5-(azulen-2-ylmethyl)-2-hydroxyphenyl]-D-glucitol

To a solution of a mixture of methyl 2,3,4,6-tetra-O-acetyl-1-C-[2-(acetyloxy)-5-(azulen-2-ylmethyl)phenyl]-D-glucopyranoside and methyl 2, 3, 4, 6-tetra-O-acetyl-1-C- [5-(azulen-2-ylmethyl)-2-hydroxyphenyl]-D-glucopyranoside in a 39:48 ratio (24.77 g and 38.91 mmol; calculated based on methyl 2,3,4,6-tetra-O-acetyl-1-C-[2-(acetyloxy)-5-(azulen-2-ylmethyl)phenyl]-D-glucopyranoside) in acetonitrile (250 ml) were added water (0.70 g), triethylsilane (18.12 g) and trimethylsilyl trifluoromethanesulfonate (26.64 g). The resulting mixture was stirred for 17 hours at -5°C. Toluene (380 ml) and a 5% aqueous sodium hydrogencarbonate solution (380 ml) were added to the reaction mixture to extract. The solvent in the organic layer was distilled off under reduced pressure. Methanol (250 ml) and a 28% sodium methoxide methanol solution (15.0 g) were added to the resulting residue, followed by stirring for 1 hour at 0°C. To the reaction mixture was added a 4 M hydrogen chloride ethyl acetate solution (20.2 g) for neutralization. The solvent in the resulting reaction mixture was distilled off under reduced pressure. Ethyl acetate (500 ml) and water (500 ml) were added to the resulting residue to extract. The organic layer was washed with water (250 ml). The aqueous layer was extracted with ethyl acetate (500 ml). The organic layer was combined with the first organic layer, and the combined organic layer was extracted with a 0.5% aqueous lithium hydroxide solution (500 ml). The organic layer was extracted three times with a 0.5% aqueous lithium hydroxide solution (250 ml). The aqueous layer extracted was neutralized with 1 M hydrochloric acid (73 ml), followed by extraction with ethyl acetate (500 ml). The aqueous layer was extracted again with ethyl acetate (250 ml). The organic layers extracted were combined and the solvent was distilled off under reduced pressure. The residue obtained was dissolved in isopropanol (17 ml) and water (17 ml). Water (34 ml) was added thereto, followed by stirring for 12 hours at room temperature. The resulting crystals deposited were collected by filtration, washed three times with an isopropanol-water (1:3) mixed solvent (6 ml) and dried under reduced pressure to obtain (1S)-1,5-anhydro-1-[5-(azulen-2-ylmethyl)-2-hydroxyphenyl]-D-glucitol [7.40 g, yield = 48%, purity 97% (HPLC)] as blue crystals.

### (Synthesis yield of compound (1) in fifth step of present invention)

The fifth step of the present invention is reduction and deprotection steps and is described specifically in Examples 7, 8 and 9. The step in prior art, corresponding to the fifth step is described in Reference Examples 5 and 6. As shown in Table 1, the synthesis yield of compound (1) in the fifth step of the present invention is 48 to 69% and, as compared with 11.5% (the synthesis yield in the Patent Document 1 (prior art)), is more than 4-fold and is improved significantly.

**Table 1**

| Yield in reduction and deprotection steps | |
|---|---|
| Fifth step of present invention | 48 to 69% |
| Patent Document 1 (prior art) | 11.5% |

### (Calculation of synthesis yield of compound (1) in fifth step of present invention)

The synthesis yield 48 to 69% of compound (1) in the fifth step of the present invention is based on that the yield in Example 7 of the present invention was 69%, the yield in Example 8 was 69% and the yield in Example 9 was 48%. The synthesis yield 11.5% of compound (1) in the Patent Document 1 (prior art) was calculated by multiplying the yield 37% in Reference Example 5 by the yield 31% in Reference Example 6, specifically, 37 x 0.31 = 11.5%.

In addition, in the Patent Document 1 (prior art), the reduction and deprotection step is carried out in two steps. That is, a reduction reaction is carried out and, after once the reduction product has been isolated, a deprotection step is carried out. In contrast, in the fifth step of the present invention, the reduction and deprotection step can be carried out continuously without isolating the reduction product; therefore, a high workability can be achieved and, moreover, high production efficiency can be achieved by the production time shortening.

### (Overall synthesis yield of compound (1) in present invention)

As shown in Table 2, the overall synthesis yield of compound (1) in the present invention is 6.2 to 9.0% and, as compared with the overall synthesis yield 1.4% in the Patent Document 1 (prior art), is 4.4-fold to 6.4-fold and is improved significantly.

**Table 2**

| Overall synthesis yield of compound (1) | |
|---|---|
| Present invention | 6.2 to 9.0% |
| Patent Document 1 (prior art) | 1.4% |

In addition, the overall synthesis yields of compound (1) in the present invention and the Patent Document 1 (prior art) were calculated as follows.

### (Calculation of overall synthesis yield of compound (1) in present invention)

The overall synthesis yield (lower limit) 6.2% of compound (1) in the present invention was calculated based on that the yield in Example 2 of the present invention was 100%, the yield in Example 3 was 26%, the yield in Example 4 was 50%, the yield in Example 6 was 100% and the yield in Example 9 was 48%. That is, the overall synthesis yield (lower limit) of compound (1) in the present invention was calculated by multiplying the yields in individual steps. Specifically, 100 x 0.26 x 0.50 x 1.00 x 0.48 = 6.2%.

Also, the overall synthesis yield (upper limit) 9.0% of compound (1) in the present invention was calculated based on that the yield in Example 2 of the present invention was 100%, the yield in Example 3 was 26%, the yield in Example 4 was 50%, the yield in Example 6 was 100% and the yield in Example 7 was 69% (or the yield in Example 8 was 69%). That is, the overall synthesis yield (upper limit) of compound (1) in the present invention was calculated by multiplying the yields in individual steps. Specifically, 100 x 0.26 x 0.50 x 1.00 x 0.69 = 9.0%.

### (Calculation of overall synthesis yield of compound (1) in Patent Document 1 (prior art))

The overall synthesis yield 1.4% of compound (1) in the Patent Document 1 (prior art) was calculated based on that the yield in Reference Example 1 was 79%, the yield in Reference Example 2 was 66%, the yield in Reference Example 3 was 61%, the yield in Reference Example 4 was 38%, the yield in Reference Example 5 was 37% and the yield in Reference Example 6 was 31%. That is, the overall synthesis yield of compound (1) in the Patent Document 1 (prior art) was calculated by multiplying the yields in individual steps. Specifically, 9 x 0.66 x 0.61 x 0.38 x 0.37 x 0.31 = 1.4%.

The structural formulas and physical and chemical properties of the compounds of above Reference Examples and above Examples are shown in Tables 3 to 5.

In addition, the symbols in each Table mean the followings.
Rf: Reference Example No.; Ex.: Example No.; STRUCTURE: structural formula; P: trimethylsilyl group or its substitute protecting group; Me: methyl; Ac: acetyl group; Et: ethyl group; Bn: benzyl group; DATA: physical property data; NMR: nuclear magnetic resonance spectrum (internal standard: TMS); MS: mass spectrometry data

**Table 3**

| Rf. | STRUCTURE | DATA |
|---|---|---|
| 1, 7 | 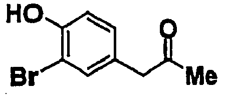 | ¹H-NMR(CDCl₃):2.17 (3H, s), 3.61 (2H, s ), 5.63 (1H, s), 6.98 (1H, d), 7.03 (1H, d d), 7.31 (1H, d) FAB-MS(m/z):230[M+H]⁺ |
| 2 | 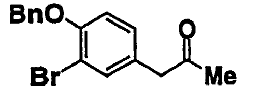 | ¹H-NMR(CDCl₃):2.16 (3H, s), 3.61 (2H, s ), 5.14 (2H, s), 6.88-7.67 (2H, dd), 7.32-7 .48 (6H, m) |
| 3 | 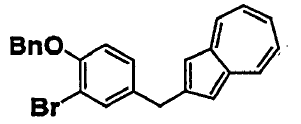 | FAB-MS(m/z):404[M+H]⁺ |
| 4 | 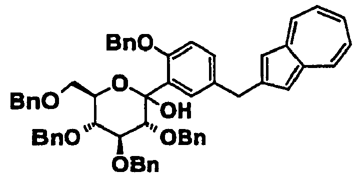 | ¹H-NMR(CDCl₃): 3.70-4.98 (19H, m), 6.8 9 (2H, d), 3.44 (3H, s), 7.08-7.63 (31H, m), 8.11 (2H, d) |
| 5 | 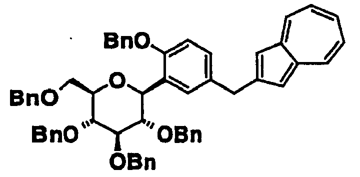 | ¹H-NMR (CDCl₃) : 3. 58-3.97 (7H, m), 4. 29-4. 99 (12H, m), 6. 85-7. 49 (33H, m), 8.0 8 (2H, d) FAB-MS(m/z):848[M+H]⁺ |
| 6 | 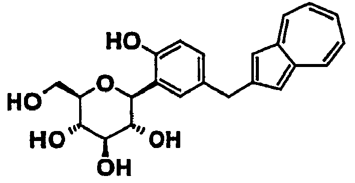 | ¹H-NMR(CD₃OD): 3.37-3.59 (4H, m), 3.70 (1H, dd), 3.82 (1H, dd), 4.23 (2H, s), 4. 56 (1H, d), 6.76 (1H, d), 7.02-7.16 (5H, m), 7.29 (1H, d); 7.49 (1H, dd), 8.17 (2H , d) FAB-MS(m/z):397 [M+H]⁺ |

**Table 4**

| Ex. | STRUCTURE | DATA |
|---|---|---|
| 1, 2 | 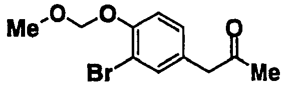 | ¹H-NMR(CDCl₃): 2.17 (3H, s), 3.52 (2H, s), 3 .62 (2H, s), 5.23 (2H, s), 7.06-7.13 (2H, m), 7.40 (1H, d) |
| 3 | 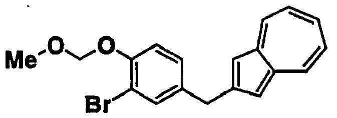 | ¹H-NMR(CDCl₃): 3.51 (3H, s), 4.26 (2H, s), 5 .21 (2H, s), 7.06-7.17 (6H, m), 7.46-7.54 (2H, m), 8.20 (2H, d) |
| 4 | 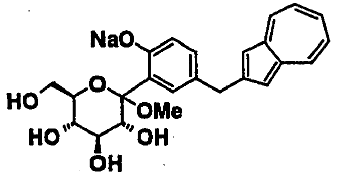 | ¹H-NMR(CD₃OD): 3.09 (3H, s), 3.30-3.32 (2H , m), 3.41-3.47 (2H, m), 3.63 (1H, ddd), 3.77 .3.92 (3H, m), 4.21 (2H, s), 6.66 (1H, d), 7.0 0 (1H, dd), 7.08-7.12 (4H, m), 7.45-7.51 (2H, m), 8.15 (2H, d) |
| 5 | 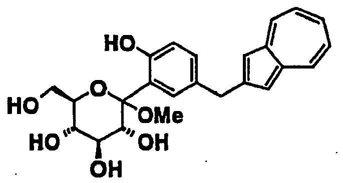 | ¹H-NMR(CD₃OD): 3.09 (3H, s), 3.30.3.32 (2H , m), 3.41-3.47 (2H, m), 3.63 (1H, ddd), 3.77 -3.92 (3H, m), 4.21 (2H, s), 6.66 (1H, d), 7.0 0 (1H, dd), 7.08-7.12 (4H, m), 7.45-7.51 (2H, m), 8.15 (2H, d) |
| 6 | 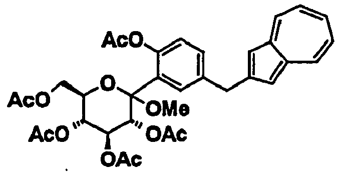 | ¹H-NMR(CDCl₃): 1.53 (3H, s), 1.94 (3H, s), 2 .04 (3H, s), 2.05 (3H, s), 2.34 (3H, s), 3.23 ( 3H, s), 4.01-4.07 (2H, m), 4.31 (2H, s), 4.43( 1H, dd), 5.17 (1H, dt), 5.27 (1H, d), 5.53(1H t), 6.99(1H, d), 7.11 (2H, s), 7.14 (2H, t), 7 .31 (1H, d), 7.32 (1H, s), 7.52 (1H, t), 8.18 ( 2H, d) FAB-MS(m/z):637[M+H]⁺ |

**Table 5**

| Ex. | STRUCTURE | DATA |
|---|---|---|
| 6 | 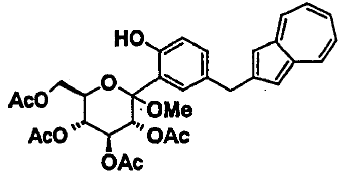 | ¹H-NMR(CDCl₃): 1.67 (3H, s), 1.95 (3H, s), 2 .05 (3H; s), 2.11 (3H, s), 3.30 (3H, s), 4.09 ( 1H, dt), 4.19 (1H, dd), 4.24 (2H, s), 4.36(1H, dd), 5.16 (1H, d), 5.29 (1H, t), 5.57(1H, t), 6.84(1H, d), 7.10-7.17 (5H, m), 7.50 (1H, t), 8.18 (2H, d) FAB-MS (m/z): 595 [M+H]⁺ |
| 7, 8, 9 | 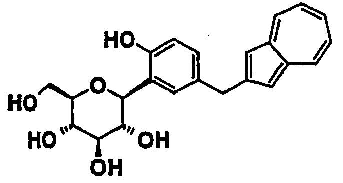 | ¹H-NMR(CD₃OD): 3.37-3.59 (4H, m), 3.70 (1 H, dd), 3.82 (1H, dd), 4.23 (2H, s), 4.56 (1H , d), 6.76 (1H, d), 7.02-7.16 (5H, m), 7.29 (1 H, d), 7.49 (1H, dd), 8.17 (2H, d) FAB-MS(m/z):397[M+H]⁺ |

### Industrial Applicability

The azulene derivative and the salt thereof, produced by the present process for producing an azulene derivative or by using an intermediate for synthesis of the azulene derivative, have the effects of inhibiting a Na⁺-glucose cotransporter and reducing the level of blood glucose; therefore, they are effective as a drug, particularly as a Na⁺-glucose cotransporter inhibitor, for treatment or prevention of, for example, insulin-dependent diabetes (type 1 diabetes), insulin-independent diabetes (type 2 diabetes), insulin-resistant diseases and obesity. In addition, the fact that the azulene derivative and the salt thereof, produced using the present invention have remarkable effects on inhibition of a Na⁺-glucose cotransporter and reduction in the level of blood glucose is confirmed in "Pharmacological Tests" (Test Examples 1 and 2) in the Patent Document 1.

## Claims

1. A process for producing an azulene derivative, **characterized by** reducing and deprotecting at least one compound selected from a compound (3) represented by the following chemical formula (1): 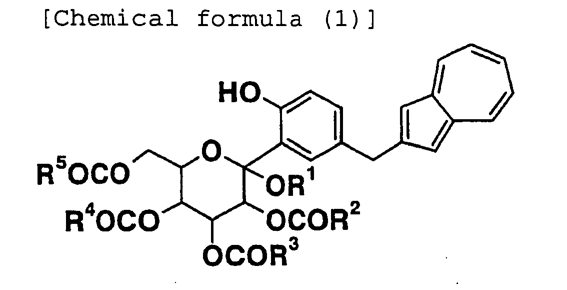 (wherein R¹ is a lower alkyl group, and R² to R⁵ may be the same or different and are each a lower alkyl group or an aryl group) or salt thereof, or at least one compound selected from compounds (2) which are each a mono-acylation product thereof, represented by the following chemical formula (2): 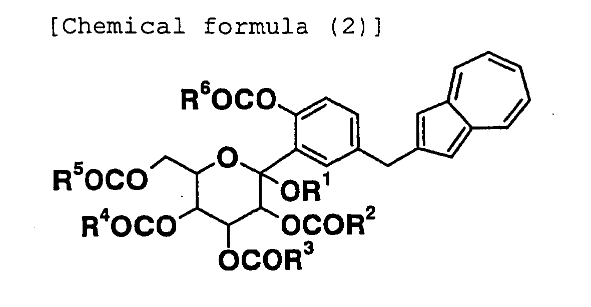 (wherein R¹ and R² to R⁵ have the same definitions as given above, and R⁵ may be the same as or different from R² to R⁵ and is a lower alkyl group or an aryl group), or a mixture of the compound (2) and the compound (3) or a mixture of a salt of the compound (2) and a salt of the compound (3), to obtain the following compound (1):

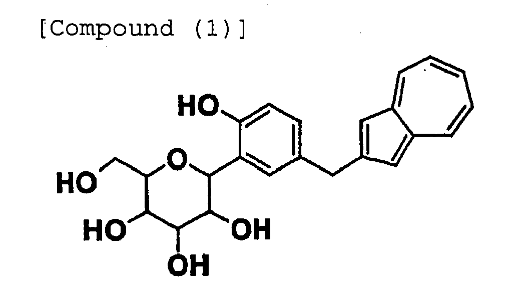

2. The process for producing an azulene derivative according to Claim 1, wherein the compound (3) represented by the chemical formula (1) contains methyl as R¹ and methyl as R² to R⁵ and is represented by the following structural formula (1) : 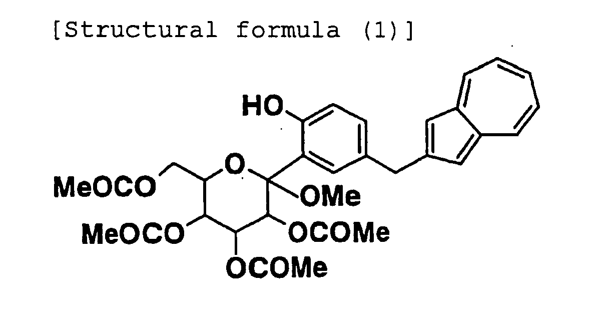 (wherein Me is methyl).

3. The process for producing an azulene derivative according to Claim 1, wherein the compound (2) represented by the chemical formula (2) contains methyl as R¹ and methyl as R² to R⁵ and R⁶ and is represented by the following structural formula (2): 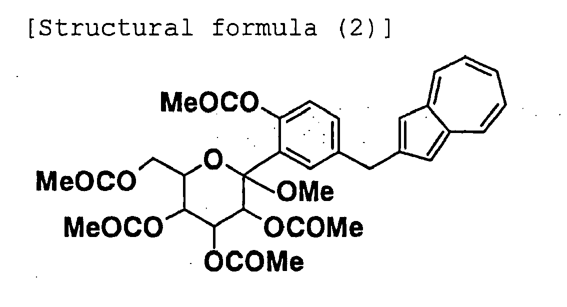 (wherein Me is methyl).

4. A process for producing an azulene derivative, **characterized by** reducing and deprotecting at least one compound selected from a compound (3') represented by the following structural formula (1): 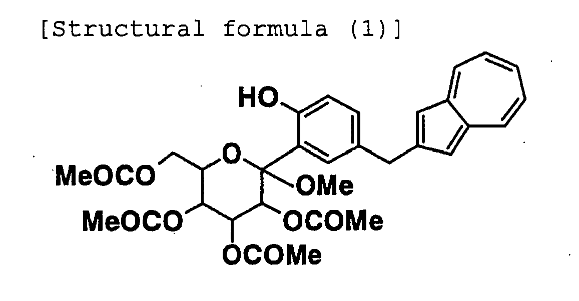 (wherein Me is methyl), or a compound (2') represented by the following structural formula (2): 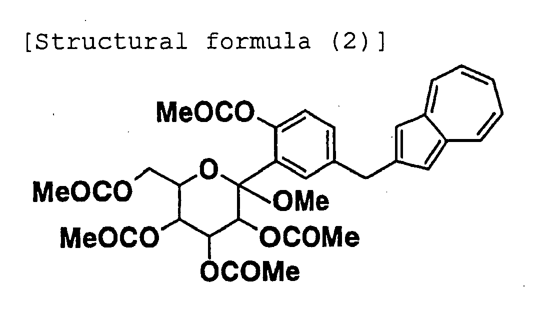 (wherein Me is methyl), or at least one compound selected from a salt of the compound (2') and a salt of the compound (3'), or a mixture of the compound (2') and the compound (3'), or a mixture of a salt of the compound (2') and a salt of the compound (3'), to obtain the following compound (1):

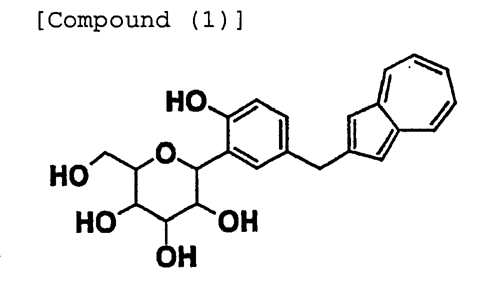

5. The process for producing an azulene derivative according to any one of Claims 1 to 4, which comprises treating the following compound (4): 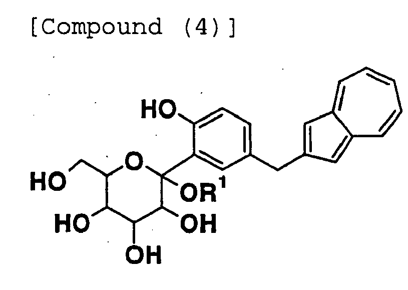 (wherein R¹ is a lower alkyl group) or a salt thereof with an acylating agent to obtain the compound (3) represented by the chemical formula (1) and/or the compound (2) represented by the chemical formula (2).

6. The process for producing an azulene derivative according to Claim 5, wherein the compound (4) contains methyl as R¹ and is a compound represented by the following structural formula (3) : 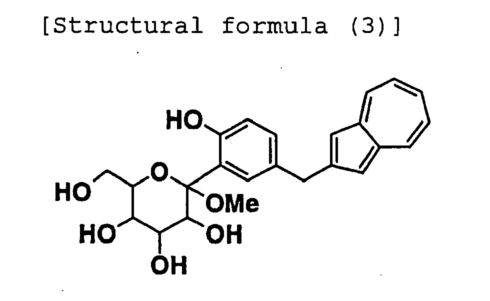 (wherein Me is methyl), and the acylating agent is an acetylating agent.

7. The process for producing an azulene derivative according to any one of Claims 1 to 6, wherein there is used, as the compound (4), a compound obtained by adding the following compound (5): 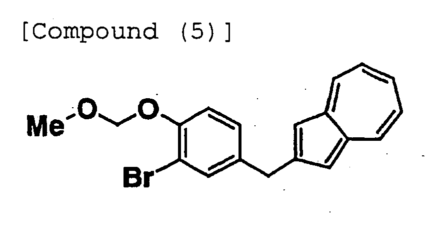 (wherein Me is methyl) to the following compound (6): 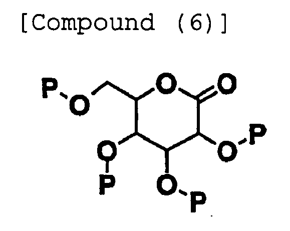 (wherein P is a trimethylsilyl group or a substitute protecting group thereof) to conduct deprotection and demethylglycosylation.

8. The process for producing an azulene derivative according to Claim 7, wherein there is used, as the compound (5), a compound obtained by reacting the following compound (7): 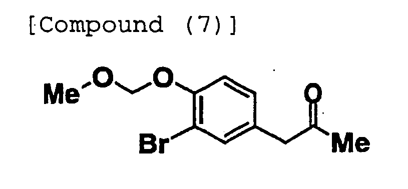 (wherein Me is methyl) with the following compound (8): 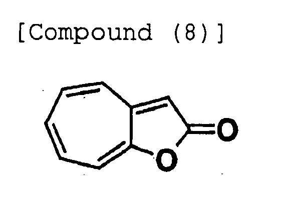 to form an azulene derivative.

9. The process for producing an azulene derivative according to Claim 8, wherein there is used, as the compound (7), a compound obtained by methoxymethylating the following compound (9): 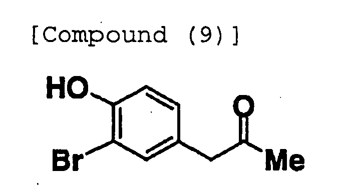 (wherein Me is methyl) or a salt thereof.

10. A compound represented by the following general formula (1): 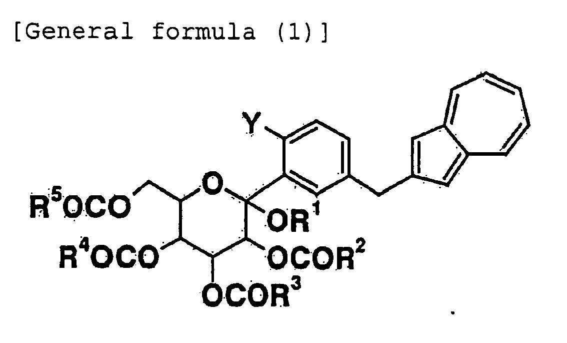 (wherein R¹ is a lower alkyl group, R² to R⁵ may be the same or different and are each a lower alkyl group or an aryl group, Y is -OH or -OCOR⁶, and R⁶ is a lower alkyl group or an aryl group) or a salt thereof.

11. The compound of general formula (1) according to Claim 10, wherein Y is -OCOR⁶ and R⁶ is a lower alkyl group or an aryl group, or a salt thereof.

12. The compound of general formula (1) according to Claim 11, which contains methyl as R¹, methyl as R² to R⁵, and methyl as R⁶ of -OCOR⁶ and is a compound represented by the following structural formula (2): 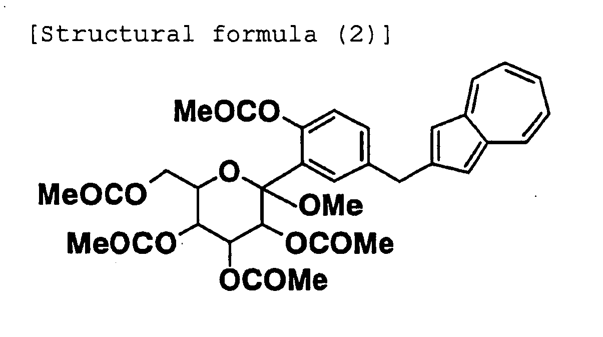 (wherein Me is methyl).

13. The compound of general formula (1) according to Claim 9, wherein Y is -OH, or a salt thereof.

14. The compound of general formula (1) according to Claim 13, which contains methyl as R¹ and methyl as R² to R⁵ and is a compound represented by the following structural formula (1) : 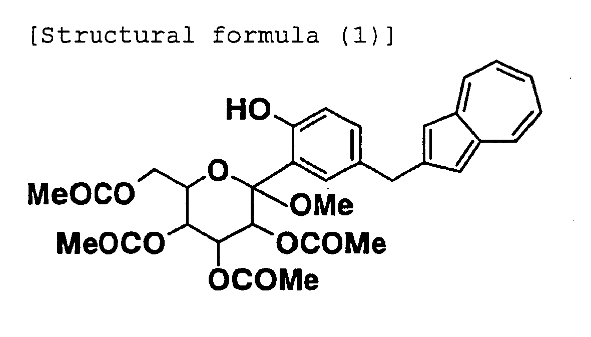 (wherein Me is methyl).

15. A compound represented by the following structural formula (3): 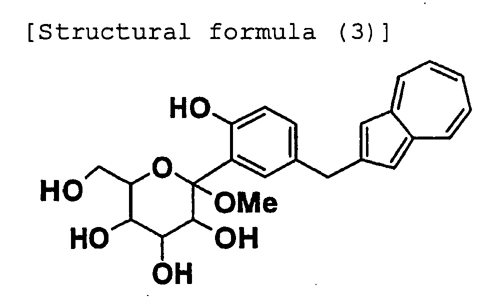 (wherein Me is methyl), or a salt thereof.

16. A compound represented by the following structural formula (4): 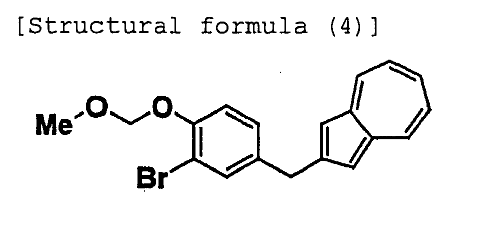 (wherein Me is methyl).

17. A compound represented by the following structural formula (5): 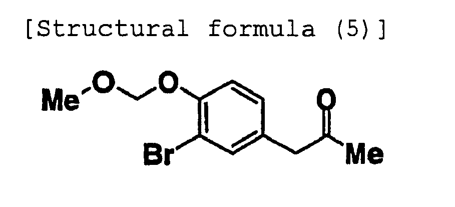 (wherein Me is methyl).
